# EUROPEAN PATENT APPLICATION

(11) **EP 1 319 660 A1**
(43) Date of publication of application: **18.06.2003**
(21) Application number: 02026925.4
(22) Date of filing: 03.12.2002
(51) Int. Cl.: C07D 453/02, C07D 205/02

(54) **Optically active azetidincarboxamide-coordinated transition metal complexes as catalysts in asymmetric reduction**

(30) Priority: 13.12.2001 JP 2001379814
(71) Applicant: Sumitomo Chemical Company, Limited, Chuo-ku Osaka 541-8550 (JP)
(72) Inventor: Taichi, Senda, Toyonaka-shi, Osaka (JP); Kazunori, Iwakura, Toyonaka-shi, Osaka (JP); Naoto, Konya, Osaka-shi, Osaka (JP)
(74) Representative: Henkel, Feiler, Hänzel

(57) **Abstract**

There are disclosed an optically active azetidincarboxamide-coordinated transition metal complex of formula (1): 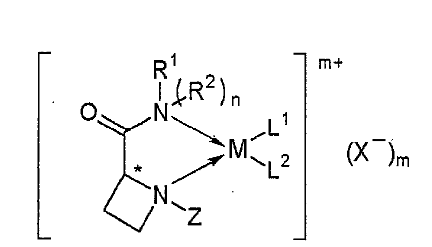 wherein R¹ and R² each independently represent hydrogen,
substituted or unsubstituted alkyl,
substituted or unsubstituted aralkyl or
substituted or unsubstituted aryl;
- Z: represents hydrogen or a protective group for amino;
- M: represents a transition metal,
- X⁻: represents a counter ion,
- m: represents an integer of 0 to 4, n represents 0 or 1, L¹ and L² each independently represent a ligand or are bonded together to represent a bidentate ligand and * indicates that the marked atom is an asymmetric carbon atom, and an azetidincarboxamide compound for producing the same.

## Description

### BACKGROUND OF THE INVENTION

### Field of the Invention

The present invention relates to optically active azetidincarboxamide-coordinated transition metal complexes, which is useful as catalysts for asymmetric synthesis using a prochiral unsaturated organic compounds, optically active azetidincarboxamide compounds and use thereof.

Asymmetric reduction of ketones using a catalyst having a proline amide compound as a ligand has been reported by Rhyoo et al (Tetrahedron Lett., 2001, vol. 42, page 5045). The report was limited to a reduction of aromatic ketones, and no description was given for the reduction of an aliphatic ketones. Azetidine pyridylamide was only known as a ligand for the nicotine acetylcholine receptor (Balboni et al. (Arzneim.-Forsch., 2000, vol. 50, page 507-511). No asymmetric catalysts using the azetidincarboxamide compounds as the ligand were known.

### SUMMARY OF THE INVENTION

According to the present invention, an optically active azetidincarboxamide-coordinated transition metal complexes of formula (1) as depicted below can be used as a catalyst in a process for producing a chiral compound from a prochiral unsaturated organic compound, using a readily available optically active azetidinecarboxylic acids.

The present invention provides
1. an optically active azetidincarboxamide-coordinated transition metal complex of formula (1): 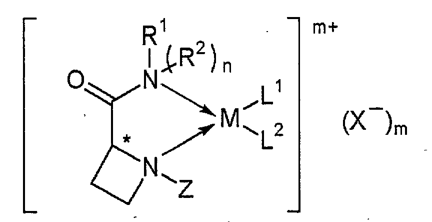 wherein R¹ and R² each independently represent hydrogen,
   substituted or unsubstituted alkyl,
   substituted or unsubstituted aralkyl or
   substituted or unsubstituted aryl;
   Z represents hydrogen or a protective group for amino;
   M represents a transition metal,
   X⁻ represents a counter ion,
   m represents an integer of 0 to 4, n represents 0 or 1,
   L¹ and L² each independently represent a ligand or are bonded together to represent a bidentate ligand and * indicates that the marked atom is an asymmetric carbon atom;
2. an optically active azetidincarboxamide compound of formula (2): 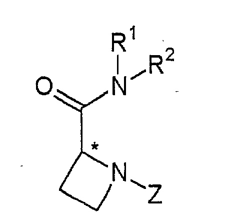 wherein R¹, R², Z and * represent the same as defined above; and
3. a method for producing an asymmetric compound from a prochiral unsaturated compound using the complex.

### DETAILED DESCRIPTION OF THE INVENTION

The invention will be described below in detail.

A description will be made to the substituent groups represented by R¹ and R² in the optically active azetidincarboxamide-coordinated transition metal complexes of formula (1) and optically active azetidincarboxamide compounds of formula (2).

Examples of the substituted or unsubstituted aryl include, for example, phenyl, tolyl, naphthyl, biphenyl, furyl, thienyl and the like.

Examples of the substituted or unsubstituted alkyl include, for example, a straight chain, branched or cyclic C1-10 alkyl group such as methyl, ethyl, n-propyl, isopropyl, n-butyl, isobutyl, t-butyl, n-amyl, neopentyl, cyclopentyl, n-hexyl, cyclohexyl, n-octyl, n-nonyl, menthyl, 2,3,4-trimethyl-3-pentyl, 2,4-dimethyl-3-pentyl and the like.

Examples of the substituted or unsubstituted aralkyl include, for example, benzyl, 2-phenylethyl, 2-naphthylethyl, diphenylmethyl and the.

These groups may be further substituted with a suitable substituent groups, and they are not particularly limited insofar as they adversely affect the invention.

Examples of the substituent include, for example, a halogen such as fluorine, chlorine, bromine or iodine;
an alkoxy (e.g. C1-4 alkoxy) such as methoxy, ethoxy, n-propoxy, t-butoxy or the like;
an aryloxy such as phenoxy and the like;
a lower alkyl(e.g. C1-6 alkyl) such as methyl, ethyl, isopropyl, n-butyl, t-butyl, n-amyl, n-hexyl and the like;
a lower alkylthio (e.g. C1-4alkylthio group) 'such as methylthio, ethylthio, n-propylthio, t-butylthio and the like;
an arylthio such as phenylthio; nitro; sulfonic acid and the like.

Examples of the protective group represented by Z or Z' include, for example, an aliphatic or aromatic acyl group(acetyl, pivaloyl, benzoyl and th elike),
a hydrocarbyloxycarbonyl group(methoxycarbonyl, ethoxycarbonyl,t-butoxycarbonyl, and the like),
a hydrocarbylsulfonyl group (methylsulfonyl, tosyl, trifuloromethylsulfonyl and th elike),
a benzyl group (e.g. benzyl, trityl, methoxybenzyl, di(p-methoxyphenyl)methyl group and the like), and
silyl group (e.g. trimethylsilyl, t-butyldimethylsilyl, diophenylmethylsilyl group and the like).

The optically active azetidincarboxamide compounds of formula (2), as the raw material in the invention, in which Z is a protective group for amino can be obtained, for example, by amidating an optically active azetidinecarboxylic acid of formula (3): 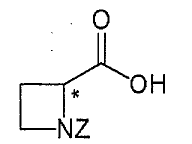 wherein Z represents a protective group for amino and * indicates an asymmetric carbon. Compounds in which Z is hydrogen can be obtained, for example, by amidating optically active azetidinecarboxylic acid of formula (4): 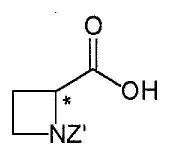 wherein Z' represents a protective group for amino and * indicates an asymmetric carbon,
and then deprotecting Z' to give the optically active azetidincarboxamide compounds of formula (2).

The amidation may be conducted in a similar manner as disclosed by Balboni et al. (Arzneim.-Forsch., 2000, vol. 50, page 509).

The protection or deprotection of the compounds of the present invention can be accomplished by introducing or removing the protecting group by similar methods as disclosed in Protective Groups in Organic Synthesis, Greene, T.W. 3^{rd} Edition, Wiley, the whole disclosure of which is incorporated herein by reference.

Examples of the transition metal represented by M preferably include rhodium, ruthenium, palladium, iridium, platinum and the like. Particularly in the asymmetric reduction reaction of the prochiral unsaturated organic compounds, rhodium and ruthenium are preferably used.

Examples of counter ion represented by X⁻ preferably include fluorine ion, chlorine ion, bromine ion, iodine ion, perchlorate, hexafluorophosphate, tetrafluoroborate, trifluoromethylbenzenesulfonate, trifluoromethanesulfonate and the like.

The ligands represented by L¹ and L² may be anyone that can be coordinated to the transition metal and include carbon monoxide, nitrogen monoxide, NH₂ and the like, as well as halogen such as chlorine, bromine and the like, olefin ligands, acetylene ligands, aromatic compound ligands,
organic oxygen-containing compound ligands,
organic sulfur-containing compound ligands,
organic nitrogen-containing compound ligands and the like.

Examples of the above-mentioned olefin ligands include, for example, ethylene, allyl, butadiene, cyclohexene, 1,3-cyclohexadiene, 1,5-cyclooctadiene, cyclooctatriene, norbornadiene, acrylic acid esters, methacrylic acid esters, cyclopentadienyl, pentamethylcyclopentadienyl and the like. In addition, 5-membered ring compounds of the following formula are can also be used as the ligand: 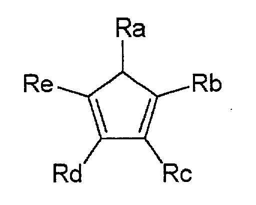 wherein Ra to Re are same or different and respectively represent hydrogen, halogen, substituted or unsubstituted alkyl, substituted or unsubstituted aralkyl, substituted or unsubstituted aryl, substituted or unsubstituted alkenyl, or substituted or unsubstituted alkoxyl or alkyloxycarbonyl group.

- Specific examples of Ra to Re include, for example, fluorine, chlorine, bromine and iodine for halogen; methyl, ethyl, n-propyl, isopropyl, n-butyl, isobutyl, t-butyl, n-amyl, neopentyl, n-hexyl, cyclohexyl, n-octyl, n-nonyl, menthyl, 2,3,4-trimethyl-3-pentyl, 2,4-dimethyl-3-pentyl and the like for the alkyl; benzyl, 2-phenylethyl, 2-naphthylethyl, diphenylmethyl and the like for aralkyl; phenyl, naphthyl, biphenyl, furyl, thienyl and the like for the aryl;
2-methyl-1-propenyl, 2-butenyl, trans-β-styryl, 3-phenyl-1-propenyl, 1-cyclohexenyl and the like for alkenyl;
methoxy, ethoxy, n-propoxy, t-butoxy and the like for alkoxyl;
phenoxy and the like for aryloxy; methoxycarbonyl, ethoxycarbonyl, t-butoxycarbonyl, benzyloxycarbonyl and the like for alkyloxycarbonyl;
phenyloxycarbonyl and the like for aryoxycarbonyl.

These groups may be further substituted with halogen as described above; alkoxyl as described above; aryloxy as described above; lower alkyl such as methyl, ethyl, isopropyl, n-butyl, t-butyl, n-amyl, n-hexyl or the like; lower alkylthio such as n-propylthio, t-butylthio or the like; arylthio such as phenylthio; nitro; or hydroxyl. The number of substituents is any number between 1 and 5 and location of substitution can be optioanlly selected.

Examples of acetylene ligands include, for example, acetylene, 1,2-dimethylacetylene, 1,4-pentadiyne, 1,2-diphenylacetylene and the like.

Examples of the aromatic compound ligands include, for excample, benzene, p-cymene, mesitylene, hexamethylbenzene, naphthalene, anthracene and the like.

Examples of the aromatic compounds typically include cyclic aromatic compounds of the following formula: 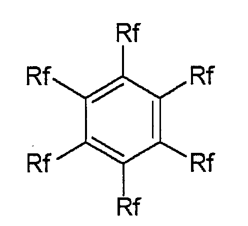 wherein Rf's are same or different and represent hydrogen, saturated or unsaturated hydrocarbon group, or heteroatom-containing functional group.

Examples of the saturated hydrocarbon include, for example, alkyl such as methyl, ethyl, propyl, isopropyl, butyl, pentyl, hexyl, heptyl and the like; and
cycloalkyl such as cyclopropyl, cyclobutyl, cyclopentyl, cyclohexyl and the like;

Examples of the unsaturated hydrocarbon include, for example, benzyl, vinyl, allyl, aryl such as a phenyl, or naphthyl group, and the-like.

Examples of the heteroatom-containing functional group include, for example, hydroxyl, alkoxyl, alkoxycarbonyl and the like. The number of substituents is any number between 1 and 6 and location of substitution is optional.

Examples of the organic oxygen-containing compound ligands include acetate, benzoate, acetylacetonate and the like.

Examples of the organic sulfur-containing compound ligands include dimethylsulfoxide, dimethylsulfide, thiophene, carbon disulfide, carbon sulfide, thiophenol and the like.

Examples of the organic nitrogen-containing compound ligands include acetonitrile, benzonitrile, t-butylisocyanide, pyridine, 1,10-phenanthroline, 2,2'-bipyridyl and the like.

Examples of the bidentate ligand include, for example, acetylacetonate, 1,3-bis(diphenylphosphino)propane, 2,2'bipyrydyl and the like.

The optically active azetidincarboxamide-coordinated transition metal complex of formula (1) can be produced by reacting an optically active azetidincarboxamide of formula (2) with a transition metal complex of the following formula (5) :

MYpLs (5)

wherein M represents a transition metal, Y represents hydrogen, halogen, carboxyl, alkoxy or hydroxy, L represents the ligand L¹ or L² as described above, and p and s respectively represent integers of 0 to 6.

Examples of the transition metal complex (5) include, for example, chlorotris(triphenylphosphine)rhodium(I), cyclopentadienylbis(triphenylphosphine)rhodium(I),
bis(cyclooctadiene)diiododirhodium(I), chloro(cyclopentadienyl)bis(triphenylphosphine)ruthenium( II), chloro(pentamethylcyclopentadienyl)(1,3-bis(diphenylphosphino)propane)ruthenium(II), chloro(pentamethylcyclopentadienyl)(1,5-cyclooctadiene)-ruthenium(II), chlorotris(triphenylphosphine)iridium(I), pentamethylcyclopentadienylbis(ethylene)iridium(I), (ethylene)bis(triphenylphosphine)platinum(0), trans-[chloro(ethyl)bis(triethylphosphine)platinum(II)], cis-[diethylbis(triethylphosphine)platinum(II)], dichloro(norbornadiene)platinum(II), tetrakis(triphenylphosphine)platinum(0), and the like. The complex that may be suitably used in the invention is not limited to these compounds.

The optically active azetidincarboxamide-coordinated transition metal complex of formula (1) can usually be produced, for example, by the following processes:

The optically active azetidincarboxamide compound of formula (2) is typically dissolved in a solvent and the transition metal complex (5) described above is added thereto. The obtained reaction solution is concentrated to give the optically active azetidincarboxamide-coordinated transition metal complex of formula (1). When the reaction product is obtained in the form of precipitates, the solid can be separated. The above procedure is usually carried out in an atmosphere of inert gas such as argon or the like. The solvent used in such reaction is not particularly limited insofar as effect of the invention is not inhibited. Examples the solvent include, for example, alcohols such as methanol, isopropanol or the like; ethers such as tetrahydrofuran, diethyl ether or the like; aromatic hydrocarbons such as toluene, benzene or the like; aliphatic hydrocarbons such as hexane, cyclohexane or the like; halogenated hydrocarbons such as dichloromethane, chloroform, chlorobenzene or the like; and so on.

Next, the process for producing asymmetric compound from a prochiral unsaturated compound using the optically active azetidincarboxamide-coordinated transition metal complex of formula (1) is described below.

The amount that may be used of the optically active azetidincarboxamide-coordinated transition metal complex of formula (1) varies depending on the reaction conditions and economy. Usually, an amount of about 1/10 to 1/100,000 mol per mol of the unsaturated organic compound as the reaction substrate can be used, and preferable amount is within a range of about 1/50 to 1/10,000.

The prochiral unsaturated organic compound includes
a ketone compound of formula (6): 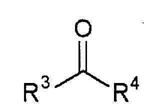 wherein R³ and R⁴ are different and represent substituted or unsubstituted alkyl, substituted or unsubstituted aryl or substituted or unsubstituted aralkyl, or R³ and R⁴ are combined together to form an asymmetric cyclic ketone, and
an imine compound of formula (7): 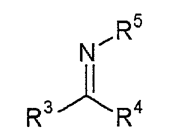 wherein R³ and R⁴ are different and represent
substituted or unsubstituted alkyl, substituted or unsubstituted aryl or substituted or unsubstituted aralkyl,
R⁵ represents hydrogen, substituted or unsubstituted alkyl, substituted or unsubstituted aryl or substituted or unsubstituted aralkyl, or
R³ and R⁴, R³ and R⁵ or R⁴ and R⁵ are bonded together to form an asymmetric cyclic imine.

The imine compound of formula (7) can be obtained by reacting an amine compound of formula: R⁵NH₂ with the ketone compound of formula (6) by known manners.

Examples of the alkyl represented by R³, R⁴ or R⁵ in the compounds of the above formulae (6) and (7) include, for example, alkyl having 1 to 8 carbon atoms (e.g. methyl, ethyl, propyl, butyl, pentyl, hexyl, heptyl, octyl or the like).

Examples of the aryl in the aryl and the aralkyl group above include, for example, phenyl, naphthyl, pyridyl, pyrimidyl, furyl, thienyl and the like.

The alkyl and aryl may be substituted with an alkyl having 1 to 6 carbon atoms (e.g. methyl, ethyl, porpyl, butyl, pentyl, hexyl or the like), an alkoxy having 1 to 6 carbon atoms (e.g. methoxy, ethoxy, porpoxy, butoxy, pentyloxy, hexyloxy or the like), halogen (e.g. fluorine, chlorine, bromine, and iodine) or the like.

The alkyl in the aralkyl includes an alkyl group having 1 to 12 carbon atoms (e.g. methyl, ethyl, propyl, butyl, pentyl, hexyl, heptyl, octyl, nonyl, decyl, undecyl, dodecyl and the like).

Examples of cyclic ketone which may be substituted of formula (6) include, for example, cycloalkenone having 5 to 12 carbon atoms, which may be fused with a benzene ring such as 1-indanone, 2-indanone, 1-tetralone, 2-tetralone, 1-benzosuberone and the like, and the cyclic ketone may be substituted with an alkyl having 1 to 6 carbon atoms (e.g. methyl, ethyl, propyl, butyl, pentyl, hexyl or the like), an alkoxy having 1 to 6 carbon atoms (e.g. methoxy, ethoxy, propoxy, butoxy, pentyloxy, hexyloxy or the like), halogen (e.g. fluorine, chlorine, bromine, and iodine) or the like.

Examples of the cyclic imine which may be substituted of formula (7) formed by combining R³ and R⁵ or R⁴ and R⁵ include 2,3,4,5-tetrahydro-6-methylpyridine, 1-methyl-3,-4-dihydroisoquinoline and the like, and the cyclic imine may be substituted with alkyl having 1 to 6 carbon atoms(e.g. methyl, ethyl, propyl, butyl, pentyl, hexyl or the like), alkoxy having 1 to 6 carbon atoms (e.g. methoxy, ethoxy, propoxy, butoxy, pentyloxy, hexyloxy or the like), halogen (e.g. fluorine, chlorine, bromine, or iodine) or the like.

Specific examples of the compounds of the formula (6) include, for example, acetophenone, propiophenone, butyrophenone, isobutyrophenone, chloromethylphenylketone, bromomethylphenylketone, 2-acetylpyridine, 3-acetylpyridine, (o-methoxy)acetophenone, (o-ethoxy)acetophenone, (o-propoxy)acetophenone, (o-benzyloxy)acetophenone, α-acetonaphthone, p-chlorophenyl methyl ketone, p-bromophenyl methyl ketone, p-cyanophenyl methyl ketone, phenyl benzyl ketone, phenyl (o-tolylmethyl) ketone, phenyl (m-tolylmethyl) ketone, phenyl (p-tolylmethyl) ketone, 2-butanone, 2-pentanone, 2-hexanone, 2-heptanone, 2-octanone, 2-nonanone, 2-decanone, cyclohexyl methyl ketone, cyclohexyl ethyl ketone, cyclohexyl benzyl ketone, t-butyl methyl ketone, 3-quinuclidinone, 1-indanone, 2-indanone, 1-tetralone, 2-tetralone, benzyl (2-pyridyl) ketone, benzyl (3-pyridyl) ketone, benzyl (2-thiazolyl) ketone and the like.

Specific examples of the compounds of the formula (7) include, for example, 3,4-dihydro-5-phenyl-1H-pyrrole, 2,3,4,5-tetrahydro-6-phenylpyridine, 1-methyl-3,4-dihydroisoquinoline, 6,7-dimethoxy-1-methyl-3,4-dihydroisoquinoline, 1-phenyl-3,4-dihydroisoquinoline, 1-methyl-3,4-dihydro-9H-pyrido[3,4-b]indole, α-methylbenzylidenebenzylamine and the like.

The ketone compound of formula (6) and the imine compound of formula (7) are asymmetrically reduced to produce a chiral organic compound such as a chiral alcohol of formula (6'): 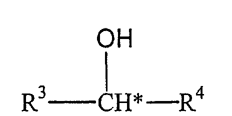 wherein R³ and R⁴ are the same as defined above and * indicates an asymmetric carbon atom, or a chiral amine of formula (7') : 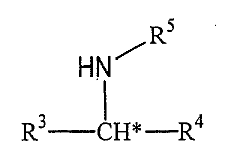 wherein R³, R⁴ and R⁵ are the same as defined above and * indicates an asymmetric carbon atom, respectively.

In a hydrogen-transfer type reduction reaction, usually a solvent is used. A solvent that solubilizes raw materials for the reaction and catalyst system is preferably used.

Additionally, a hydrogen source is required in the reaction. Examples of the hydrogen source include, for example, a primary or secondary alcohol, an unsaturated compound such as cyclohexene, cyclohexadiene, indoline and the like, formic acid and salts thereof are utilized. Preferred are secondary alcohols (e.g. isopropanol, sec-butanol, cyclohexanol, 1-phenethyl alcohol and the like) and unsaturated compounds, and more preferred are secondary alcohols.

The reaction temperature is usually -90 to 150°C and preferably the reaction can be carried out at about -20 to 100°C.

The reaction period varies depending on conditions such as concentration of the substrate, temperature and the like and usually the reaction is completed within several hours to about 30 hours. After completion of the reaction the product may be separated or isolated in a known manner such as extraction, phase-separation, distillation and/or chromatography, if necessary.

In addition, the reaction in the invention can be carried out either by batch system or by continuous system as the reaction mode.

Preferred azetidincarboxamide compounds are an azetidincarboxamide compound of formula (2), wherein either one of R¹ and R² is hydrogen, more preferred is an azetidincarboxamide compound of formula(2), wherein R² is hydrogen and R¹ is substituted or unsubstituted alkyl, substituted or unsubstituted phenyl or substituted or unsubstituted naphthyl, and furthermore preferred is an azetidincarboxamide compound of formula (2), wherein z is hydrogen in the more preferred compound above.

Specific examples of the optically active azetidincarboxamide compound of formula (2) of the present invention include, for example, optically active N-phenyl-2-azetidinecarboxamide, N-(1-naphthyl)-2-azetidinecarboxamide, N-(4-methoxyphenyl)-2-azetidinecarboxamide, N-(2-fluorophenyl)-2-azetidinecarboxamide and the like.

Azetidincarboxamide compounds of formula (2) wherein R² is a phenyl group, Z is a hydrogen atom, and R¹ is a methyl group or a hydrogen atom were erroneously disclosed in Table 1, page 504 by Balboni et al. (Arzneim.-Forsch., 2000, vol. 50, page 507-511) despite the fact that the disclosed compounds should have a pyridyl group in place of the phenyl group as R² group in the above-described compounds.

The optically active azetidincarboxamide-coordinated transition metal complexes of formula (1) include transition metal complexes derived from the above optically active azetidincarboxamide compound of formula (2) and the like.

### EFFECT OF THE INVENTION

The optically active azetidincarboxamide-coordinated transition metal complex of formula (1) of the invention is useful as a catalyst for producing chiral compound from a prochiral unsaturated organic compounds.

### EXAMPLES

The invention is described below in more detail with reference to Examples. These Examples should not be considered as a limitation upon the invention.

### Example 1

Under an argon atmosphere, 1.00 g (4.97 mmol) of (S)-N-t-butoxycarbonyl-2-azetidinecarboxylic acid was dissolved in 15 ml of tetrahydrofuran and 502 mg (4.97 mmol) of triethylamine was added thereto. While stirring the reaction mixture at 0°C, 539 mg (4.97 mmol) of ethyl chlorocarbonate was added gradually and dropwise. After the dropping was completed, the mixture was stirred at 0°C for 30 minutes, and then 463 mg (4.97 mmol) of aniline was added. After stirring at 0°C for 1 hour, the stirring was continued at room temperature for 16 hours. The reaction mixture was stirred under heating to reflux for further 3 hours, and then cooled to room temperature. Solid was filtered and washed with ethyl acetate. Then the organic layer was concentrated and the residue was purified by column chromatography to give the desired product, i.e., optically active 1.27 g (yield: 93%) of (S)-1-t-butoxycarbonyl-N-phenyl-2-azetidinecarboxamide in the form of a white solid.

Into 5 ml of chloroform was dissolved 494 mg (1.79 mmol) of (S)-1-t-butoxycarbonyl-N-phenyl-2-azetidinecarboxamide obtained above, and 2 ml of trifluoroacetic acid was gradually dropped at room temperature. After the dropping was completed, the mixture was stirred at room temperature for 2 hours. Then 20 ml of toluene was added and the mixture was concentrated in a rotary evaporator. The residue was adjusted to pH 7 by addition of 0.1M methanolic sodium hydroxide solution and the obtained solution was concentrated. The residue was dissolved in 20 ml of 1N hydrochloric acid and washed with diethyl ether. The washed residue was made alkaline with sodium hydroxide and extracted with 20 ml of diethyl ether. The obtained organic layer was washed with a saturated sodium hydroxide solution, dried over anhydrous sodium sulfate and concentrated to give 234 mg (yield: 74%) of (S)-N-phenyl-2-azetidinecarboxamide in the form of a white solid. ¹H-NMR (heavy chloroform, δ(ppm)): 2.17 (s, 1H), 2.40-2.50 (m, 1H), 2.67-2.73 (m, 1H), 3.32-3.39 (m, 1H), 3.82 (q, J=8.4 Hz, 1H), 4.42 (t, J=8.4 Hz, 1H), 7.11 (t, J=8.4 Hz, 1H), 7.34 (t, J=8.4 Hz, 2H), 7.63 (d, J=8.4 Hz, 2H), 9.55 (s, 1H). ¹³C-NMR (heavy chloroform, δ(ppm)): 27.66, 44.46, 60.60, 120.37, 125.18, 130.07, 138.79, 173.24.

### Example 2

The procedure for reaction and post-treatment in Example 1 was repeated except that 1-naphthylamine was used in place of aniline to give (S)-N-(1-naphthyl)-2-azetidinecarboxamide. Overall yield: 69%.
¹H-NMR (heavy chloroform, δ(ppm)): 2.22 (s, 1H) , 2.50-2.57 (m, 1H), 2.75-2.80 (m, 1H), 3.43-3.50 (m, 1H), 3.88 (q, J=8.5 Hz, 1H), 4.55 (t, J=8.5 Hz, 1H), 7.46-8.29 (m, 7H), 10.40 (s, 1H). ¹³C-NMR (heavy chloroform, δ(ppm)): 27.15, 43.96, 60.39, 118.28, 120.61, 125.07, 125.22, 126.27, 126.41, 126.53, 129.21, 132.71, 134.45, 172.71.

### Example 3

The procedure for reaction and post-treatment in Example 1 was repeated except that 4-methoxyaniline was used in place of aniline to give (S)-N-(4-methoxyphenyl)-2-azetidinecarboxamide. Overall yield: 63%.
¹H-NMR (heavy chloroform, δ(ppm)): 2.17 (s, 1H), 2.40-2.47 (m, 1H), 2.67-2.72 (m, 1H), 3.32-3.38 (m, 1H), 3.80 (s, 3H), 3.78-3.86 (m, 1H), 4.42 (t, J=8.5 Hz, 1H), 6.88 (d, J=9.0 Hz, 2H), 7.55 (d, J=9.0 Hz, 2H), 9.43 (s, 1H). ¹³C-NMR (heavy chloroform, δ(ppm)): 26.98, 43.76, 55.86, 59.84, 114.52, 121.26, 131.36, 156.58, 172.23.

### Example 4

The procedure for reaction and post-treatment in Example 1 was repeated except that 2-fluoroaniline was used in place of aniline to give (S)-N-(2-fluorophenyl)-2-azetidinecarboxamide. Overall yield: 61%.
¹H-NMR (heavy chloroform, δ(ppm)): 2.30 (s, 1H), 2.42-2.51 (m, 1H), 2.68-2.73 (m, 1H), 3.35-3.41 (m, 1H), 3.82 (q, J=8.5 Hz, 1H), 4.46 (t, J=8.5 Hz, 1H), 7.02-7.16 (m, 3H), 8.38-8.46(m, 1H), 9.90 (s, 1H). ¹³C-NMR (heavy chloroform, δ(ppm)): 26.94, 43.80, 60.00, 115.22 (d, J_{CF}=19.0 Hz), 121.41, 124.53 (d, J_{CF}=7.5 Hz), 124.87 (d, J_{CF}=3.7 Hz), 126.64 (d, J_{CF}=10.3 Hz), 153.01 (d, J_{CF}=244.0 Hz), 172.76.

### Example 5

Under an argon atmosphere, 215 mg (1.22 mmol) of (S)-N-phenyl-2-azetidinecarboxamide obtained in Example 1 was dissolved in 7 ml of anhydrous isopropanol and 337 mg (0.55 mmol) of dichlororuthenium (p-cymene) complex [RuCl₂(p-cymene)]₂ was added thereto. Then, 246 mg (2.44 mmol) of triethylamine was added. The obtained mixture was stirred with heating at 80°C for 1 hour. Then, the solution was concentrated and anhydrous diethyl ether was added to the residue to give (S)-N-phenyl-2-azetidinecarboxamide chlororuthenium (p-cymene) complex in the form of yellowish orange powders in a quantitative yield. MS (EI, m/e): 446, 410, 234, 134.

### Example 6

Under an argon atmosphere, 2.4 mg (0.005 mmol) of (S)-N-phenyl-2-azetidinecarboxamide chlororuthenium (p-cymene) complex obtained in Example 5 and 62.6 mg (0.5 mmol) of 3-quinuclidinone were dissolved in 2 ml of isopropanol. To the obtained solution was added 20 µl (0.01 mmol) of 0.5M potassium hydroxide solution in isopropanol and allowed to react at 0°C for 6 hours. After the reaction was completed, The reaction mixture was concentrated and the residue was purified by silica gel column chromatography to give the desired product, i.e., optically active 3-quinuclidinol. The analysis of the optical purity of the product with HPLC having an optically active stationary phase showed a purity of 64% ee. The conversion was 99%.

### Example 7

The procedure in Example 6 was repeated except that (S)-N-(1-naphthyl)-2-azetidinecarboxamide chlororuthenium (p-cymene) complex (0.005 mmol) was used as the catalyst and the reaction temperature was changed to 30°C to give optically active 3-quinuclidinol (optical purity: 23%ee). The conversion was 99%.

### Example 8

The procedure in Example 6 was repeated except that (S)-N-(4-methoxyphenyl)-2-azetidinecarboxamide chlororuthenium (p-cymene) complex (0.005 mmol) was used as the catalyst and the reaction temperature was changed to 30°C to give optically active 3-quinuclidinol (optical purity: 52%ee). The conversion was 99%.

### Example 9

The procedure in Example 6 was repeated except that (S)-N-(2-fluorophenyl)-2-azetidinecarboxamide chlororuthenium (p-cymene) complex (0.005 mmol) was used as the catalyst and the reaction temperature was changed to 30°C to give optically active 3-quinuclidinol (optical purity: 46%ee). The conversion was 99%.

### Example 10

The procedure in Example 6 was repeated except that acetophenone (0.5 mmol) was used in place of 3-quinuclidinone and the reaction temperature was changed to 30°C to give optically active phenylethanol (optical purity: 68%ee). The conversion was 94%.

## Claims

1. An optically active azetidincarboxamide-coordinated transition metal complex of formula (1): 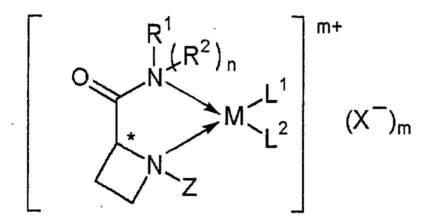 wherein R¹ and R² each independently represent hydrogen,
substituted or unsubstituted alkyl,
substituted or unsubstituted aralkyl or
substituted or unsubstituted aryl;
Z represents hydrogen or a protective group for amino;
M represents a transition metal,
X⁻ represents a counter ion,
m represents an integer of 0 to 4, n represents 0 or 1, L¹ and L² each independently represent a ligand or are bonded together to represent a bidentate ligand and * indicates that the marked atom is an asymmetric carbon atom.

2. An optically active azetidincarboxamide compound of formula (2): 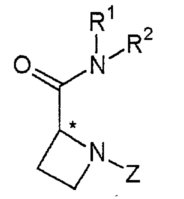 wherein R¹ and R² each independently represent hydrogen,
substituted or unsubstituted alkyl,
substituted or unsubstituted aralkyl or
substituted or unsubstituted aryl;
Z represents hydrogen or a protective group for amino; and * indicates an asymmetric carbon.

3. The optically active azetidincarboxamide compound according to claim 2, wherein either one of R¹ and R² is hydrogen.

4. The optically active azetidincarboxamide compound according to claim 2 or 3, wherein R² is hydrogen and R¹ is substituted or unsubstituted alkyl, substituted or unsubstituted phenyl or substituted or unsubstituted naphthyl.

5. The optically active azetidincarboxamide compound according to claim 2 or 3, wherein Z is hydrogen.

6. The optically active azetidincarboxamide compound according to claim 4, wherein Z is hydrogen.

7. A process for asymmetrically reducing a prochiral unsaturated organic compound, which comprises hydrogenating a prochiral unsaturated organic compound in the presence of the optically active azetidincarboxamide-coordinated transition metal complex of claim 1, thereby producing a corresponding chiral organic compound.

8. The process according to claim 7, wherein the unsaturated organic compound is a ketone compound or an imine compound.

9. The process according to claim 7 or 8, wherein the reaction is conducted in the presence of a hydrogen source.

10. The process according to claim 9, wherein the hydrogen source is a secondary alcohol.

11. The process according to claim 10, wherein the secondary alcohol is isopropanol.
